# EUROPEAN PATENT SPECIFICATION

(11) **EP 2 572 638 B1**
(45) Date of publication and mention of the grant of the patent: **26.07.2023**
(21) Application number: 11842125.4
(22) Date of filing: 15.11.2011
(51) Int. Cl.: G01R 33/563, A61B 5/055

(54) **MAGNETIC RESONANCE IMAGING APPARATUS AND MAGNETIC RESONANCE IMAGING METHOD**
MAGNETRESONANZTOMOGRAPHIEVORRICHTUNG UND MAGNETRESONANZTOMOGRAPHIEVERFAHREN
APPAREIL D'IMAGERIE PAR RÉSONNANCE MAGNÉTIQUE ET PROCÉDÉ D'IMAGERIE PAR RÉSONANCE MAGNÉTIQUE

(30) Priority: 15.11.2010 US 946549
(43) Date of publication of application: 27.03.2013
(73) Proprietor: Toshiba Medical Systems Corporation, Otawara-shi, Tochigi-ken 324-0036 (JP)
(72) Inventor: MIYAZAKI, Mitsue, Otawara-shi Tochigi 324-8550 (JP)
(74) Representative: Marks & Clerk LLP
(86) International application number: PCT/JP2011/076328
(87) International publication number: WO 2012/067123

(56) References cited:
- JP-A- 2001 149 340
- JP-A- 2004 024 637
- JP-A- 2004 024 637
- JP-A- 2007 312 966
- TAKAHASHI J ET AL: "Optimization of non-contrast renal MRA using a TI-prep scan for time-spatial labeling pulse (time-SLIP) in 3D balanced SSFP", INTERNATIONAL SOCIETY FOR MAGNETIC RESONANCE IN MEDICINE. SCIENTIFIC MEETING AND EXHIBITION. PROCEEDINGS, INTERNATIONAL SOCIETY FOR MAGNETIC RESONANCE IN MEDICINE, US, 19 April 2008 (2008-04-19), page 2903, XP002614073, ISSN: 1524-6965
- MIYAZAKI MITSUE ET AL: "Nonenhanced MR angiography", RADIOLOGY, RADIOLOGICAL SOCIETY OF NORTH AMERICA, INC, US, vol. 248, no. 1, 1 July 2008 (2008-07-01), pages 20-43, XP002670571, ISSN: 0033-8419, DOI: 10.1148/RADIOL.2481071497
- YIU-CHO CHUNG ET AL: "Inversion Recovery Cine TrueFISP for Optimizing TI in Mycardial Infarct Imaging", PROCEEDINGS OF THE INTERNATIONAL SOCIETY FOR MAGNETIC RESONANCE IN MEDICINE, ISMRM, 10TH SCIENTIFIC MEETING AND EXHIBITION, HONOLULU, HAWAII, USA, 18-24 MAY 2002, 4 May 2002 (2002-05-04), XP040584952,
- MITSUE MIYAZAKI ET AL.: 'Nonenhanced MR Angiography' RADIOLOGY vol. 248, no. 1, July 2008, pages 20 - 43, XP002670571

## Description

### Field

Exemplary embodiments of the present invention relate to a magnetic resonance imaging apparatus and a magnetic resonance imaging method. Background

Magnetic resonance imaging (MRI) is an imaging method in which nucleus spins of a subject placed on a static magnetic field is magnetically excited by using a high frequency (RF) signal of a Larmor frequency and an image is reconstructed from a magnetic resonance (MR) signal generated in accordance with the excitation. In the filed of magnetic resonance imaging, non-contrast MRA (magnetic resonance angiography) is known as a method of obtaining blood flow images without using a contrast agent.

### Citation List

### Patent Literature

JP 2004 024637 relates to an MRI apparatus for obtaining an MRI image by an ECG (electrocardiography) gated IR method which comprises: a test photographing means for collecting an MRI image by applying a plurality of TI values set in advance; a means for selecting an optimal image from a plurality of sheets of image sensitivity obtained by this photographing means and obtaining the optimal TI value corresponding to this optimal image: and a main photographing means for performing main photographing by applying this optimal TI value as a photographing condition. Chung et al "Inversion Recovery Cine TrueFISP for Optimising TI in Myocardial Infarct Imaging" in proceedings of the International Society for magnetic resonance in medicine, 10 scientific meeting and exhibition, 18 to 24 May 2002 relates to a study proposing a new sequence that provides a better choice of TI for inversion recovery based infarct imaging independent of sequences used.

### Non-Patent Literature

Takahashi et al "optimisation of non-contrast renal MR a using a Tl-prep scan the time-spatial labelling pulse (time-SLIP) in 3-D balanced SSFP" in "International Society for Magnetic Resonance in medicine, scientific meeting and exhibition, proceedings, 19 April 2008 page 2903 relates to a technique where a time-slip pulse was placed to mark the aorta, which flows directly into the renal arteries. Miyazaki et al, "Non-enhanced MR angiography" in radiology, Radiological Society of North America, Inc, volume 248, July 2008 pages 20 to 43 is an article reviewing the state-of-the-art in non-enhanced MR angiography. This article refers to, amongst many techniques, ASL angiography which relies upon the spin tagging upstream of arteries of interest by using an inversion pulse to generate image contrast.

A problem to be solved by the invention is to acquire appropriate images of the blood flow in a cardiac muscle tissue.

### Brief Description of Drawings

FIG. 1 is a schematic block diagram of an MRI system according to an exemplary embodiment;
FIG. 2 is a chart illustrating a time-SLIP (spatial labeling inversion pulse) sequence which can be used when performing the present invention;
FIG. 3 is a flowchart depicting a procedure of a computer program code executed in the MRI system according to the exemplary embodiment, wherein the procedure is only encompassed by the claims when step S07 shown in Fig. 3 is carried out;
FIG. 4 is a diagram illustrating cine images which were acquired in accordance with an exemplary embodiment;
FIG. 5 is a diagram illustrating the initial, segmented time-SLIP sequence according to the exemplary embodiment;
FIG. 6 is a diagram illustrating cine images which were acquired using the initial, segmented time-SLIP sequence according to the exemplary embodiment;
FIG. 7 is a diagram illustrating another example of the initial, segmented time-SLIP sequence according to the exemplary embodiment;
FIG. 8 is a diagram illustrating another example of the initial, segmented time-SLIP sequence according to the exemplary embodiment;
FIG. 9A is a diagram illustrating the relationship between a labeling area and an imaging area according to the exemplary embodiment; and
FIG. 9B is a diagram illustrating the relationship between the labeling area and the imaging area according to the exemplary embodiment.

### Description of Embodiments

The invention provides a magnetic resonance imaging apparatus according to claim 1 and a magnetic resonance imaging method according to claim 9.

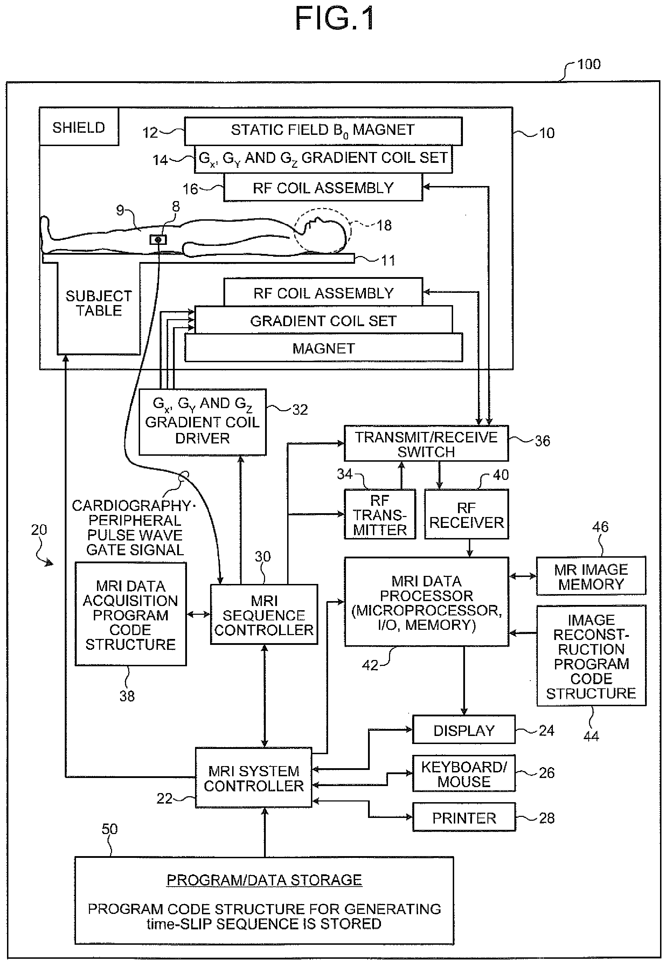

The MRI (magnetic resonance imaging) system 100 shown in FIG. 1 includes a gantry 10 (shown in cross-section) and various related system components 20 interfaced therewith. At least the gantry 10 is typically provided in a shielded room. One MRI system 100 depicted in FIG. 1 includes a substantially coaxial cylindrical arrangement of a static field B₀ magnet 12, a Gₓ, G_{y} and G_{z} gradient coil set 14 and an RF (radio frequency) coil assembly 16. Along the horizontal axis of these cylindrically-arrayed elements, there is an imaging volume 18 shown as encompassing the head of a subject 9 supported by a subject table 11.

An MRI system controller 22 has input/output ports connected to a display 24, a keyboard/mouse 26 and a printer 28. As will be appreciated, the display 24 may be of the touch-screen variety so that it provides control inputs as well.

The MRI system controller 22 interfaces with an MRI sequence controller 30. The MRI sequence controller 30 controls Gₓ, G_{y} and G_{z} gradient coil drivers 32, as well as an RF transmitter 34 and a transmit/receive switch 36 (if the same RF coil is used for both transmission and reception). As those in the art will appreciate, one or more electrodes 8 may be affixed to the subject to provide ECG (electrocardiogram) signals and/or peripheral pulse wave gating signals to the MRI sequence controller 30. The MRI sequence controller 30 has access to suitable program code structure 38 for executing available pulse sequences to generate MR images by using operator inputs and/or system inputs defining particular sequence parameters.

The MRI system 100 includes an RF receiver 40 providing input to an MRI data processor 42 so as to create processed image data to be output to the display 24. The MRI data processor 42 may be configured for access to an image reconstruction program code structure 44 and to a MR image memory 46 (e.g., for storing MRI data derived from processing in accordance with the exemplary embodiments and the image reconstruction program code structure 44).

FIG. 1 also gives a generalized depiction of a MRI system program/data storage 50. Program code structures stored in the MRI system program/data storage 50 (for generation of time-SLIP images and cine images, operator inputs for the generation, etc.) are stored in computer-readable storage media accessible to the various data processing components of the MRI system. As those in the art will appreciate, the program storage 50 may be segmented and directly connected, at least in part, to various ones of the processing computers of the system 20 having most immediate need for program code structures stored as described above in their normal operation (i.e., rather than being commonly stored in and connected directly to the MRI system controller 22).

Indeed, as those in the art will appreciate, the depiction of FIG. 1 is a very high-level simplified diagram of a typical MRI system with some modifications so as to practice exemplary embodiments to be described hereinbelow. The system components can be divided into various logical collections of "boxes" and typically include numerous digital signal processors (DSP), microprocessors, special purpose processing circuits (e.g., for fast A/D conversions, fast Fourier transforming, array processing, etc.). Each of those processors is typically a clocked "state machine" wherein the physical data processing circuits progress from one physical state to another upon the occurrence of each clock cycle (or predetermined number of clock cycles).

Not only does the physical state of processing circuits (e.g., CPUs (central processing units), registers, buffers, arithmetic units, etc.) progressively change from one clock cycle to another during the operation, the physical state of associated data storage media (e.g., bit storage in magnetic storage media) is transformed from one state to another during operation of such a system. For example, at the conclusion of an MRI reconstruction process, an array of computer-readable accessible data value storage sites in physical storage media will be transformed from some prior states (e.g., all uniform "zero" values or all "one" values) to a new state. In such a new state, the physical states at the physical sites of such an array vary between minimum and maximum values to represent real world physical events and conditions (e.g., the tissues of a subject over an imaged volume space). As those in the art will appreciate, such arrays of stored data values represent and also constitute a physical structure. In other words, when such arrays are sequentially loaded into instruction registers and executed by one or more CPUs of the MRI system 100, a particular sequence of operational states occurs and a particular structure of computer control program codes transferred into the MRI system 100 is structured.

The exemplary embodiments described below provide improved ways to acquire and/or process data and generate and display MR images.

As MRI techniques, ASL (arterial spin labeling) for assessing myocardial blood flow without using a contrast agent is known. ASL is an imaging method of obtaining perfusion images by magnetically labeling the blood with RF pulses and using the labeled blood as a tracer. For example, the MRI system acquires control images for which the labeled blood does not contribute, acquires tagged images for which the labeled blood contributes by applying inversion pulses as an example of labeling pulses, and performs a difference processing on the acquired images. The tissues made still by the difference processing are canceled, so that the MRI system can obtain only images of blood components flowing into the imaging area. Regarding ASL, see, for example Zun, et al., "Assessment of Myocardial Blood Flow (MBF) in Humans Using Arterial Spin Labeling (ASL): Feasibility and Noise Analysis," Magnetic Resonance in Medicine, 62:975-983 (2009). Here, a two-dimensional ASL sequence is used to examine the tag on/off signal difference in the myocardium (with and without stress perfusion).

In addition, a time-SLIP (spatial labeling inversion pulse) method is known as MRI techniques. The time-SLIP technique is an imaging method of depicting a fluid flowing into or flowing out of an imaging area by labeling the fluid in a position independent of the imaging area and increasing or reducing the signal value of the fluid flowing into or flowing out of the imaging area. For example, the MRI system applies a time-SLIP pulse after a given wait time interval from a synchronization signal (e.g., R wave). The Time-SLIP pulses include an area non-selective inversion pulse and an area selective inversion pulse and on or off can be set for the area non-selective inversion pulse. When the fluid flowing into (or flowing out of) the imaging area is labeled by using the area selective inversion pulse, the intensity of the signal of the part that the fluid reaches after BBTI (black-blood time to inversion) time interval increases (decreases when the area non-selective inversion pulse is off). Regarding this time-SLIP technique, see, for example, US 2011/0071382 A1. In this related application, a non-contrast cardiac perfusion time-SLIP technique is used to observe tagged (or marked) blood flow distribution by selecting an appropriate BBTI time interval.

Here, selection of an appropriate BBTI parameter value (hereinafter also referred to as "BBTI value" or "BBTI delay interval") is critical in the time-SLIP technique. This is because, if the selected BBTI value is not appropriate, the "tagged" blood flow bolus may not reach the imaged ROI (region of interest) or may have already passed the ROI by the time MR data is acquired. Selecting an appropriate BBTI parameter value is difficult. This is because the BBTI value varies from subject to subject and varies even for the same subject depending upon heart rate and/or other subject-specific conditions.

With the angiography using the time-SLIP technique incorporating a variably positionable cine sub-sequence, the MRI system 100 according to the invention can alleviate and/or eliminate the problem in that selecting an appropriate BBTI value is difficult. Specifically, in order to find a myocardial perfusion signal difference in a high temporal resolution, the MRI system 100 according to the invention uses a cine sequence (e.g. a 2D cine sequence) to examine the signal intensity changes of the myocardium during desired cardiac phases (e.g., diastole). To examine signal changes in cardiac phases using flow-out, the MRI system 100 can use 2D time-SLIP technique with balanced SSFP (steady state free precession).

In particular, in an exemplary embodiment incorporating a cine sub-sequence (e.g., bSSFP or FFE (fast field echo) of two dimensions (e.g., one spatial dimension and one time dimension) or three dimensions (e.g., two spatial dimensions and one time dimension)), the MRI system 100 can discretionarily select a range of BBTI values for a given pulse sequence. For example, the duration of a segmented cine sub-sequence incorporated within the pulse sequence using the time-SLIP technique (hereinafter, time-SLIP sequence) can be controlled (e.g., by the operator or the MRI system 100). Moreover, according to the invention, the recovery time after the cine sub-sequence (i.e. the recovery time before a new data acquisition cycle) and any desired initial triggering delay (i.e. any delay subsequent to a cardiac triggering event selected before one or more RF tagging RF pulses are applied) is controlled.

After acquiring data using the variably positionable cine sub-sequence in a data acquisition cycle in the time-SLIP sequence, the MRI system 100 according to the exemplary embodiment can automatically perform any required image subtraction processing or other processing/analysis of acquired signals (e.g., with alignment between images). Preferably, RF tag "on" and tag "off" parameters can be preselected by the operator (or system) so that the MRI system 100 can thereafter automatically perform multiple pulse sequences before performing necessary image differencing operations.

The MRI system 100 according to the exemplary embodiment eventually can provide indications of blood perfusion signal intensity changes within the imaged myocardium that is displayed and/or stored and/or exported to a remote system or a remote site. The MRI system 100 provides an identified one of the MRI cine image frames acquired using the time-SLIP sequence incorporating the cine sub-sequence. Because another time-SLIP sequence is determined by previously identifying the most appropriate MRI cine image frame, another time-SLIP sequence is executed by using an appropriate BBTI value.

A time-SLIP sequence incorporating a cine sub-sequence may include, for example, the following cases: (a) one area-selective inversion pulse is applied upstream of the ROI so as to effect a flow-in angiography, or (b) an area non-selective inversion pulse and an area-selective inversion pulse are applied to the ROI so as to effect a flow-out angiography. Alternately setting on/off of the area selective inversion pulse makes subtraction for outputting the blood vessel easy.

In a time-SLIP sequence according to the invention, there are three time dimensions that can be controlled (namely an initial triggering delay, the duration of a segmented cine sub-sequence, and a recovery time). On the other hand, in scanning where the area selective inversion pulse is alternately turned on/off, only the initial triggering delay and the duration of the cine sub-sequence may be selectable (e.g., by an operator and/or MRI system programming). It is preferable that the alignment between multiple images of the same ROI be carefully performed (e.g., so as to compensate for motion artifacts between cine image frames).

By using time-SLIP sequence with an incorporated segmented cine sub-sequence, the MRI system 100 according to the invention can depict signal changes during the subject's cardiac cycle (regardless whether the flow-in angiography with only a single area selective inversion pulse is used or a flow-out angiography with an area selective inversion pulse and an area non-selective inversion pulse are used). In the flow-in angiography, the MRI system 100 applies an area selective inversion pulse to the myocardium. The myocardium signal strength then decreases with increasing BBTI time interval during a time-SLIP sequence. In the flow-out angiographic approach, an area selective inversion pulse and an area non-selective inversion pulse are applied to an upstream portion of the myocardium. The myocardium signal strength then increases with increasing BBTI during a time-SLIP sequence. In the method of alternately setting on/off the area selective inversion pulse (hereinafter, "on/off alternate approach"), the myocardium signal strength increases with increasing BBTI time interval. The tag on/tag off alternate approach helps to eliminate background signals and observe blood flow into the myocardium.

The MRI system 100 according to the invention obtains the BBTI value in the cine sub-sequence with the efficient signal intensity change as an efficient BBTI value candidate possibly used in the subsequent time-SLIP sequence. The subsequent time-SLIP sequence is a time-SLIP sequence not incorporating a cine sub-sequence (a different desired sequence is used). In addition, the MRI system 100 according to the exemplary embodiment can use a cine image frame (that can be used to determine the most optimum BBTI value) as a myocardial perfusion image.

For example, as an initial time-SLIP sequence incorporating a cine sub-sequence, the MRI system 100 performs relatively quick and efficient 2D time-SLIP sequence so as to identify the appropriate BBTI value. Thereafter, the MRI system 100 according to the exemplary embodiment performs a lengthy 3D time-SLIP sequence not incorporating a cine sub-sequence for multiple times over multiple slices so as to depict a three-dimensional image of the myocardium.

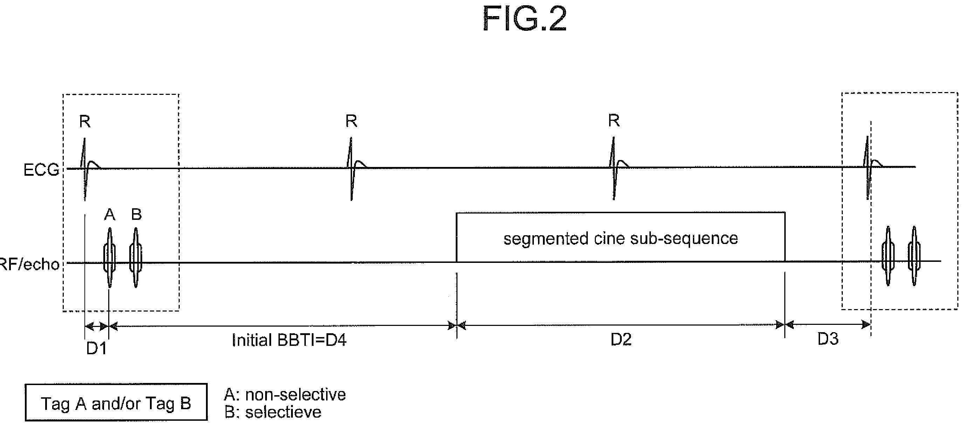

FIG. 2 is a chart illustrating a time-SLIP sequence incorporating a variably positionable cine sub-sequence. The upper line in FIG. 2 depicts the R-wave of the subject's ECG (electrocardiogram) signal. As shown in FIG. 2, the MRI system 100 according to the invention applies inversion pulses A and/or B (e.g., 180° pulses), which are labeling RF pulses, after an initial trigger delay D1. The initial triggering delay D1 is usually short and may be close to zero. The gradient pulses are not shown explicitly in FIG. 2 to simplify the drawing. In addition, as shown in FIG. 2, the MRI system 100 according to the invention continues acquiring data by using the segmented cine sub-sequence during the duration D2. For example, the MRI system 100 according to the exemplary embodiment depicts 4 to 5 cine image frames effectively taken at sequential BBTI time intervals by continuing acquiring data by using the cine sub-sequence in a state where an RF pulse, SS (slice select) pulse, PE (phase encode) pulse, and RO (read out) pulse, etc are preferably initialized as having at least about 300-400 milliseconds covering at least the subject's diastole.

As depicted in FIG. 2, the initial BBTI delay interval D4 (e.g., approximately 600 milliseconds) is the delay from the initial triggering delay and is positioned as the beginning of the segmented cine sub-sequence. After the segmented cine sub-sequence and a further recovery time delay D3, the MRI system 100 according to the invention starts a subsequent data acquisition cycle (e.g., to allow the nuclear magnetization to return to a suitable starting condition).

In an exemplary embodiment, the operator or the MRI system 100 can select these time intervals such that they conform to a given subject's ECG signal. For example, the operator or the MRI system 100 may efficiently define the time intervals in the entire single sequence by selecting all or any three of D1, D2, D3 and D4. Alternatively, the operator or the MRI system 100 may define other time intervals as, for example, the initial setting value defined for the MRI system 100. In the exemplary embodiment, the operator or the MRI system 100 can previously choose or preset whether to use the inversion pulse A and/or the inversion pulse B. When the MRI system 100 according to the exemplary embodiment uses the area non-selective inversion pulse A and the area selectable inversion pulse B, it can depict selectively-marked, or tagged, blood flow into the myocardium.

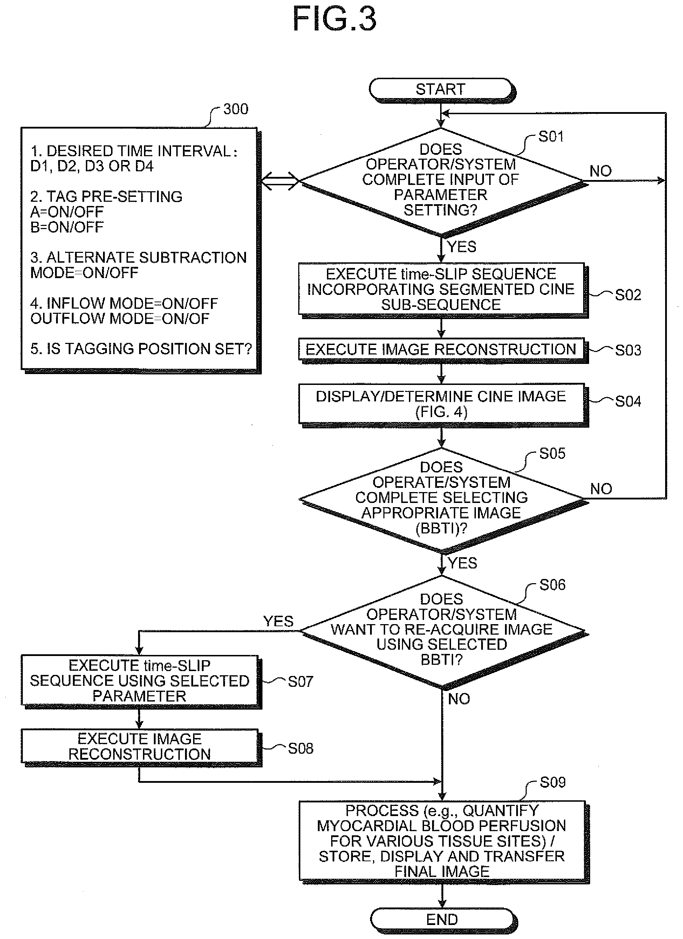

FIG. 3 is a flowchart depicting a procedure of a computer program code executed by the MRI system 100 according to the exemplary embodiment. However, the procedure shown in Fig. 3 is only encompassed by the claims if step S07 is carried out. The MRI system 100 first determines whether the operator or the MRI system 100 has completed inputting a parameter setting (step S01). For example, the operator input is as represented by a GUI (graphical user interface) 300. For example, in the exemplary embodiment, the operator can select some of the time intervals D1, D2, D3 and D4 at step S01. The operator may select only one, two or three of these intervals, while the MRI system 100 may determine the remaining interval(s). If the length of the total time interval is known and, when there are n time intervals, then the operator or the MRI system 100 defines n-1 of the time intervals. The operator or the MRI system 100 can independently define tagging presets for both inversion pulses A and B, if desired (i.e., the operator or the MRI system 100 can presets on/off of each of the inversion pulses A and B). The operator or the MRI system 100 can also define on on/off alternate approach. The operator or the MRI system 100 can alternatively select the flow-in mode or the flow-out mode so as to alternatively select the tagging position (e.g., upstream of the ROI or within the ROI). Some or all of these pulse sequence parameters may be selected by the MRI system 100 and/or selected by the operator via a suitable GUI.

When the pulse sequence parameter is defined at step S01 (YES step S01), the MRI system 100 executes the time-SLIP sequence incorporating the segmented cine sub-sequence so as to acquire MR data (step S02). Suitable cine sub-sequences for use as the cine sub-sequence are known, per se. See, for example, US Patent Application Publication No. 2008/0061780 A1 (filed September 10, 2007 and published March 13, 2008) and/or US Patent Application Publication No. 2011/0074416 A1 filed March 12, 2010. Such cine sequences may also be referred to as segmented cine sequences (because such cine sequences are segmented in k-space).

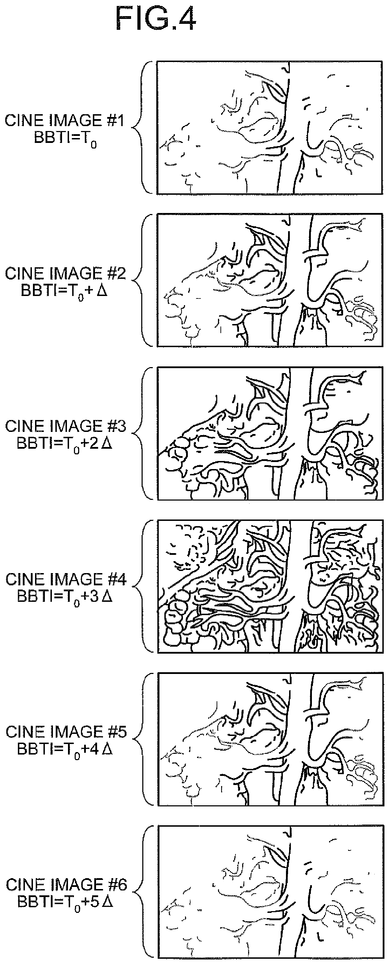

The MRI system 100 then executes a suitable image reconstruction processing (step S03). This image reconstruction processing includes alignment between images and the image subtraction process after the alignment. The MRI system 100 according to the exemplary embodiment then displays the acquired cine image frames as shown in FIG. 4. As is clear from FIG. 4, Frame 4 is the most clear among the successive cine images. Accordingly, the operator or the MRI system 100 can identify the BBTI time interval associated with that frame as an appropriate BBTI time interval. The identifying of an appropriate cine image frame and its corresponding BBTI time interval may be effected also by suitably-programmed computer processes without displaying the images to the operator (as is also indicated at step S04 in FIG. 3). At step S05, the MRI system 100 determines whether the operator or the MRI system 100 has identified an allowable and appropriate BBTI time interval (step S05). If not (NO at step S05), the MRI system 100 returns to step S01 to allow the operator or the MRI system 100 to input a parameter setting.

If an appropriate image and a BBTI interval has been identified (YES at step S05), the MRI system 100 determines whether there is an instruction from the operator or the MRI system 100 to, by using the identified BBTI time interval (e.g., possibly use of a 3D series of multi-slice data acquisition cycles may be desired), re-acquire an MRA image using a time-SLIP sequence (step S06). When it is determined to acquire MRA images using a time-SLIP sequence (YES at step S06), the MRI system 100 executes the time-SLIP sequence with the chosen parameters including the identified appropriate BBTI parameter (step S07). According to the invention, step S07 is always carried out. The MRI system 100 then executes image reconstruction, including required image subtraction processing and using known and preferable image alignment, for the MR data acquired at step S07 (step S08). In an example outside the scope of the invention as defined by the claims, when it is determined to acquire no MRA image using the time-SLIP sequence at step S06 (NO at step S06), the MRI system 100 (which has already used the appropriate BBTI value identified in fact) outputs the pre-chosen cine image frame as a final output image (step S09).

Regardless whether the final output image is the chosen cine image or the MRA image acquired with the new time-SLIP sequence by using the appropriate BBTI value, the MRI system 100 further processes the MRA image of the appropriate time-SLIP sequence. Alternatively, the MRI system 100 performs all or any of storing and displaying of the MRA image of the appropriate time SLIP sequence as well as transferring/exporting the MRA image to a remote facility/process/system (e.g., the MRI system 100 subtracts, by pixel-by-pixel basis, the magnitude of pixel values in one image from the magnitude of corresponding pixel values in another image and/or subtracts complex-valued pixels in one image from corresponding complex-valued pixels in another image so as to suitably quantify the myocardial blood flow for various tissue sites in accordance with procedures already known by those skilled in the art).

The MRI system 100 according to the invention will be described again below. The MRI system 100 according to the invention includes an MRI sequence controller configured to acquire an ECG signal from a subject, a first imaging execution unit, an identifying unit, and a second imaging execution unit. After applying a labeling RF pulse to the blood flowing into the myocardium of the subject, the first imaging execution unit performs a segmented cine sub-sequence of an imaging area including the myocardium. Accordingly, the first imaging execution unit acquires multiple non-contrast MR data sets for which the time intervals between labeling and acquiring data are different. The identifying unit identifies, on the basis of the acquired multiple MR data sets acquired by the first imaging execution unit, a time interval taken by the labeled blood to reach a given position in the imaging area. The second imaging execution unit sets the identified time interval as a corresponding parameter of a further time-SLIP sequence. After applying a labeling RF pulse to the blood flowing into the myocardium of the subject, the second imaging execution unit acquires non-contrast MR data by imaging the imaging area including the myocardium. For example, the MRI system controller 22 shown in FIG. 1 includes the first imaging execution unit, the identifying unit, and the second imaging execution unit (not shown) and, by controlling each unit of the MRI system, the above-described various functions can be effected.

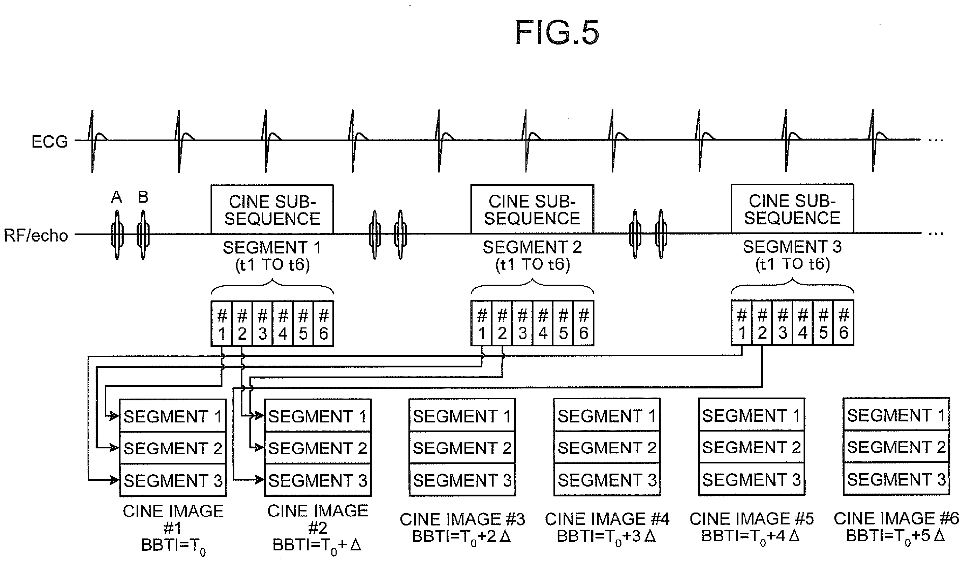

The function of each unit will be described in detail using specific examples. First, according to the invention, the first imaging execution unit performs a time-SLIP sequence incorporating a segmented cine sub-sequence. FIG. 5 is a diagram illustrating the time-SLIP sequence according to the exemplary embodiment. As shown in FIG. 5, after a given delay has passed from the trigger regarding the cardiac phase (i.e. R-wave) of the subject, the first imaging execution unit applies inversion pulses A and B (or only an inversion pulse B).

Subsequently, the first imaging execution unit executes the cine sub-sequence using a segment k-space method in a given duration (denoted by the rectangle of "cine sub-sequence"). The segment k-space method is a data acquisition method of segmenting the k-space into multiple segments and sequentially acquiring k-space data in each segment. For example, it is assumed that the k-space is segmented into three segments as shown in FIG. 5. The first imaging execution unit acquires multiple MR data sets for which the BBTI time intervals are different with respect to Segment 1. The MR data acquired is k-space data of Segment 1 corresponding to cine images #1 to #6 as shown in FIG. 5. The first imaging execution unit acquires multiple MR data sets for which BTTI time intervals are different with respect to Segment 2 Furthermore, the first imaging execution unit acquires multiple MR data sets for which BBTI time intervals are different with respect to Segment 3. As described above, the first imaging execution unit acquires MR data sets for which BBTI time intervals are different with respect to every segment over multiple heart rates. In the exemplary embodiment, the first imaging execution unit performs the segmented cine sub-sequence during a given time interval including the diastole among the subject's cardiac phase.

On the basis of the multiple MR data sets acquired by the first execution unit, the identifying unit identifies a time interval taken by the labeled blood to reach a given position in the imaging area. Some methods can be applied to the identifying process performed by the identifying unit. Methods 1 to 3 described below are nothing more than examples.

In Method 1, for example, the identifying unit generates multiple MRA (magnetic resonance angiography) images from the acquired MR data. In other words, the identifying unit reconstructs the multiple MR data acquired with respect to each segment and for which BBTI time intervals are different by consolidating them with respect to each BBTI value so that multiple MRA images for which BBTI time intervals are different are generated. In this case, because the identifying unit consolidates the MR data acquired over multiple heart rates into a single MRA image, it is preferable that alignment be performed. The identifying unit outputs the generated multiple MRA images to the display 24 and receives an input specifying a given MRA image from the operator.

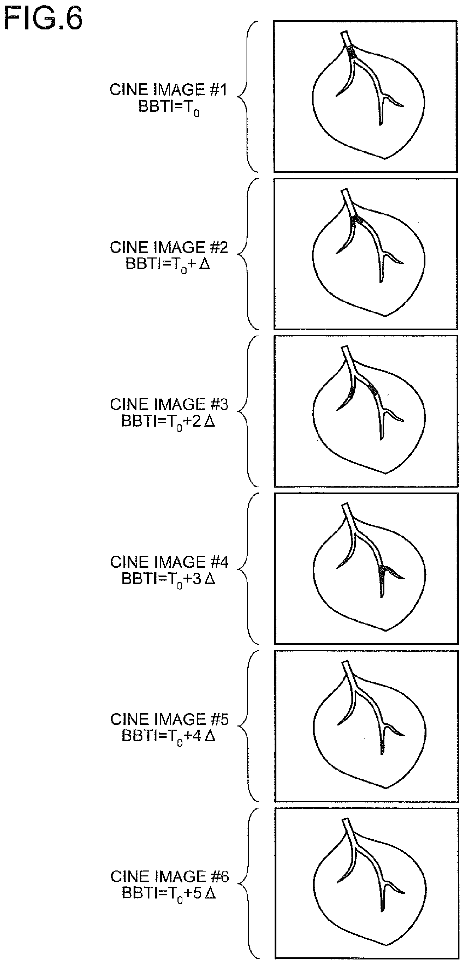

FIG. 6 is a diagram illustrating cine images which were acquired using the segmented time-SLIP sequence according to the exemplary embodiment. For example, as shown in FIG. 6, the identifying unit outputs multiple MRA images for which BBTI time intervals are different (cine images #1 to #6) to the display 24. The operator then determines, for example, that Cine image #4 is an appropriate MRA image and makes an input specifying Cine image #4. The identifying unit receives the input and identifies, as a time interval taken by the labeled blood to reach the given position in the imaging area, a BBTI time interval (BBTI=T₀+3Δ) corresponding to the specified MRA image. The "given position in the imaging area" is, for example, the position that a doctor who gives a diagnosis wants to actually observe and it can be set arbitrarily.

In Method 2, for example, the identifying unit analyzes signal values of the acquired multiple MR data and automatically identifies, on the basis of the result of the analysis results of the analyzed signal values, a time interval taken by the labeled blood to reach a given position in an imaging area. For example, after generating multiple MRA images for which BBTI time intervals are different, the identifying unit analyzes signal values of an area in each of the MRA images (for example, corresponding to the given position in the imaging area). The identifying unit determines the image with the highest signal value (or the image with lowest signal value) in the area and identifies the BBTI time interval corresponding to the image as a time interval taken by the labeled blood to reach the given position in an imaging area.

In Method 3, for example, the identifying unit analyzes the signal values of acquired multiple MR data and outputs the analysis result of the analyzed signal values to the display 24. For example, after generating multiple MRA images for which BBTI time intervals are different, the identifying unit analyzes signal values of an area in each of the MRA images (for example, corresponding to the given position in the imaging area). The identifying unit then outputs a graph depicting the pixel value transition in combination with the corresponding index of the MRA image. The operator then, for example, performs an input specifying the MRA image with the highest signal value. The identifying unit receives this input and identifies, as the time interval taken by the labeled blood to reach a given position in the imaging area, the BBTI time interval corresponding to the specified MRA image.

When the time interval taken by the labeled blood to reach a given position in the imaging area is identified, the second imaging execution unit sets the time interval identified by the identifying unit as the BBTI value that is a parameter of a normal time-SLIP sequence not incorporating any cine sub-sequence. The second imaging execution unit executes this time-SLIP sequence and acquires MR data. For example, the first imaging execution unit acquires two-dimensional MR data and the second imaging execution unit acquires three-dimensional MR data.

As described above, in the MRI system 100 according to the invention, the first imaging execution unit performs imaging in order to apply an appropriate parameter (BBTI value) to imaging performed by the second imaging execution unit. According to the invention, because of the high temporal resolution of imaging performed by the first imaging execution unit, an appropriate parameter (BBTI value) can be selected and applied to the imaging performed by the second imaging execution unit. In other words, because the first imaging execution unit acquires MR data by using the segmented cine sub-sequences, the imaging temporal resolution is high. Circulation of an order of few msec to few tens of msec can be performed.

The MRI system 100 according to the invention receives a setting, made by the operator, of various parameters regarding the time-SLIP sequence executed by the first imaging execution unit or the parameters are automatically determined in the MRI system 100. In other words, the MRI system 100 according to the exemplary embodiment further includes a receiver that receives a setting of a parameter regarding the given time interval and the first imaging execution unit positions the given time interval on the time axis of the pulse sequence in accordance with the received setting of the parameter and executes the segmented cine sub-sequence. For example, the MRI system controller 22 shown in FIG. 1 includes the receiver (not shown).

For example, the receiver receives a setting of at least one of D1, D2, D3 and D4 described above using FIG. 2. As shown in FIG. 2, D1 is the time interval between the trigger (R-wave) regarding the subject's heart rate and application of the first RF pulse (inversion pulse A or inversion pulse B). D2 is the time interval of the given time interval. D3 is the time interval between the end of the given time interval and the start of the next cycle. D4 is the time interval between the first RF pulse (the inversion pulse A or inversion pulse B) and the start of the given time interval.

The reason why D3 is set will be described. As shown in FIG. 5, the time-SLIP sequence executed by the first imaging execution unit repeatedly executes a cycle including a given time interval (denoted by the rectangle of "cine sub-sequence" in FIG. 5). As described below, one MRA image is generated by consolidating each segment acquired in each cycle. Because the cardiac cycle of the subject is not necessarily constant, if MR data in each segment is acquired on the basis of only the elapsed time from the trigger (i.e. R-wave) regarding the subject's heart phase, for example, inconsistency occurs in the recovery of the vertical magnetic components between segments. Accordingly, the image quality of the MRA image generated by consolidating each segment also decreases. For this reason, according to the invention, D3 that should be secured as a recovery time is set (or as an elapsed time after application of an inversion pulse). According to the invention, when the next R-wave is detected before D3 is not secured yet, the first imaging execution unit does not acquire any MR data and waits until the next cycle. In contrast, when the next R-wave is detected after D3 is secured, the first imaging execution unit acquires MR data.

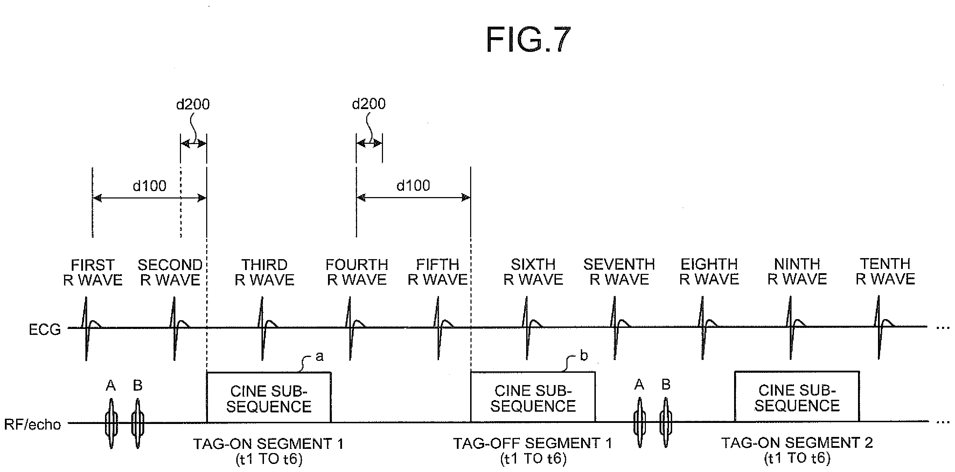

FIG. 7 is a diagram illustrating another example of the segmented time-SLIP sequence according to the exemplary embodiment. For example, the first imaging execution unit may alternately turn on/off the inversion pulse as shown in FIG. 7. In this case, the first imaging execution unit applies an inversion pulse at a heart rate and then acquires multiple MR data for which BBTI time intervals are different with respect to Segment 1. The MR data acquired here is, like the case shown in FIG. 5, k-space data of Segment 1 corresponding to Cine images #1 to #6. The first imaging execution unit then, without applying any inversion pulse in another heart rate, acquires multiple MR data for which BBTI time intervals are different with respect to the same Segment 1. Thereafter, the first imaging execution unit next applies an inversion pulse at another heart rate and acquires multiple MR data for which BBTI time intervals are different with respect to Segment 2. As described above, the first imaging execution unit may alternately perform acquisition in which an inversion pulse is applied and acquisition in which no inversion pulse is applied.

A supplementary description will be given for the relationship between acquisition in which an inversion pulse is applied and acquisition in which no inversion pulse is applied. In the example of FIG. 7, the time interval between a first R wave and the start of a cine sub-sequence a in which acquisition is performed by applying an inversion pulse regarding Segment 1 is equal to the time interval between a fourth R-wave and the start of a cine sub-sequence b in which acquisition is performed regarding Segment 1 without applying any inversion pulse (d100 in FIG. 7). However, the exemplary embodiment is not limited to this. Because no inversion pulse is applied in the cine subsequence b, for example, the time interval between a second R-wave and the start of a cine sub-sequence may be equal to the time interval between the fourth R-wave and the start of the cine sub-sequence b (d200 in FIG. 7). In other words, in this case, the cine sub-sequence b begins after the time d200 has passed after the fourth R-wave.

The exemplary embedment is not limited to a technique of alternately performing acquisition in which an inversion pulse is applied and acquisition in which no inversion pulse is applied. For example, for one image, acquisition in which no inversion pulse is applied may be collectively performed and then acquisition in which an inversion pulse is applied may be sequentially performed. For example, after acquisition in which no inversion pulse is applied is performed regarding Segments 1 to 3, acquisition in which an inversion pulse is applied may be successively performed with respect to each of the segments. Acquisition in which no inversion pulse is applied for one image is not necessarily performed at first, and it may be performed at desired timing (intermediate timing or last).

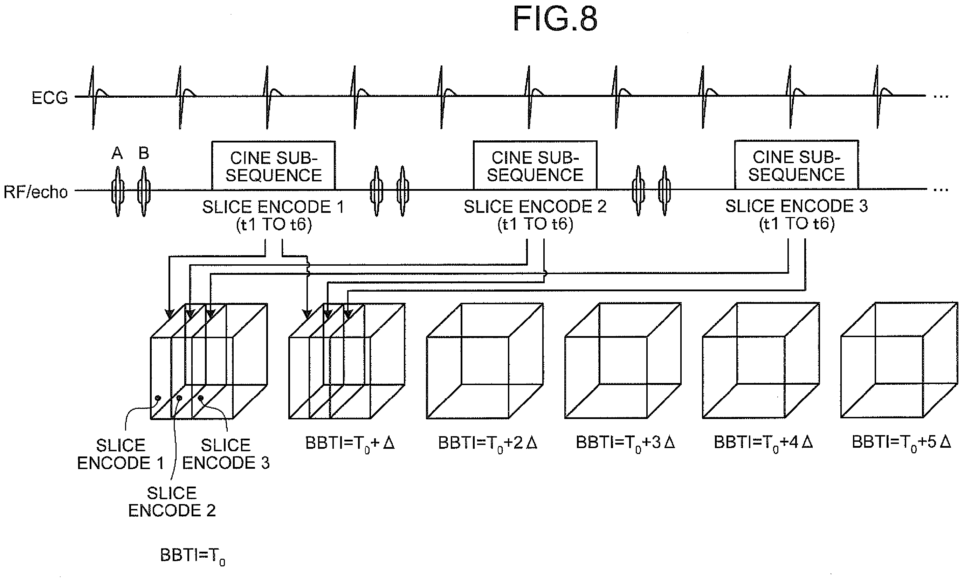

FIG. 8 is a diagram illustrating another example of the segmented time-SLIP sequence according to the exemplary embodiment. For example, as shown in FIG. 8, the first imaging execution unit may acquire multiple three-dimensional data for which BBTI time intervals are different. For example, the first imaging execution unit acquires, at a heart rate, multiple MR data sets for which BBTI time intervals are different regarding Slice Encode 1 that is a segment of the three-dimensional data. The first imaging acquisition unit acquires, at another heart rate, multiple MR data sets for which BBTI time intervals are different regarding Slice Encode 2. Although illustrations are appropriately omitted in FIG. 8, the first imaging execution unit acquires multiple MR data sets for which BBTI time intervals are different for cardiac phases over multiple heart rates for every slice encode.

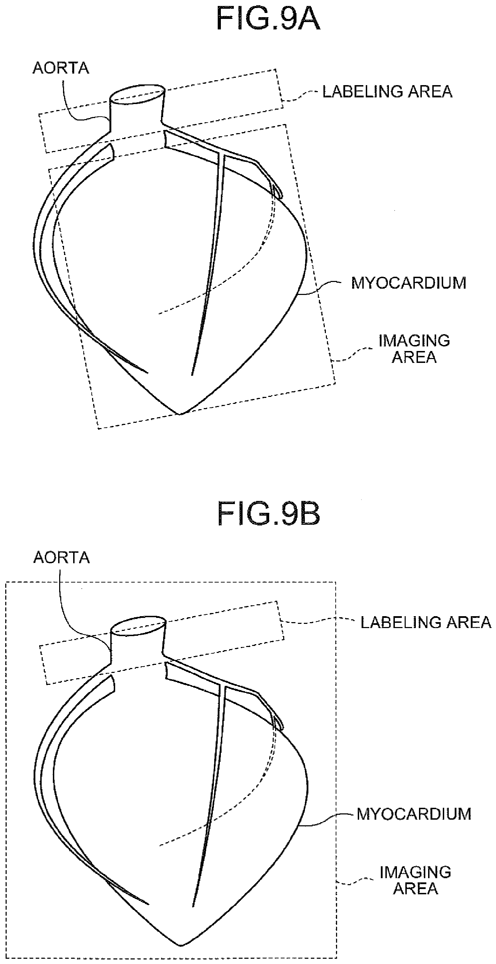

Furthermore, the relationship between a labeling area and an imaging area according to the exemplary embodiment will be described below. FIGS. 9A and 9B are diagrams illustrating the relationship between a labeling area and an imaging area according to the exemplary embodiment.

FIG. 9A is a diagram illustrating flow-in angiography. In the exemplary embodiment, in the case of flow-in angiography, an inversion pulse A, which is an area non-selective inversion pulse, is not applied but only an inversion pulse B, which is an area selective inversion pulse, is applied. The inversion pulse B is applied to a labeling area that is set outside the imaging area and independent of the imaging area. As shown in FIG. 9A, the labeling area is set in the aorta positioned upstream of the myocardium. Application of an inversion pulse B to the labeling area inverts the vertical magnetic components of the blood in the labeling area. Accordingly, the blood in the labeling area is labeled and, in the imaging area, the signal value of the part that the blood reaches after the BBTI time interval decreases.

FIG. 9B is a diagram illustrating flow-out angiography. In the exemplary embodiment, in the case of flow-out angiography, both of an inversion pulse A, which is an area non-selective inversion pulse, and an inversion pulse B, which is an area selective inversion pulse, are applied. The inversion pulse A is applied all over the imaging area. Because the inversion pulse A is applied all over the imaging area, the vertical magnetic components of the myocardium and blood in the labeling area are inverted. The inversion pulse B is then applied to the labeling area that is set outside the imaging area and independent of the imaging area. As shown in FIG. 9B, the labeling area is set in the aorta positioned upstream of the myocardium. Application of the inversion pulse B to the labeling area selectively inverts the vertical magnetic components of the blood in the labeling area after the recovery in the time interval between the inversion pulse A and the inversion pulse B. In this manner, the blood in the labeling area is labeled and, in the imaging area, the signal value of the part that the blood reaches after the BBTI time interval increases.

The definitions of flow-in angiography and flow-out angiography are not limited to the above-described definitions. By some definitions, they may be referred to as the opposite names or other names. Setting of an imaging area and a labeling area may be arbitrarily changed in accordance with the purpose of imaging. Furthermore, as application examples, the labeling method may be, for example, pCASL (pulsed continuous arterial spin labeling) in which an inversion pulse is successively applied.

According to the magnetic resonance imaging apparatus and the magnetic resonance imaging method according to at least one of the above-described exemplary embodiments, appropriate images of the blood flow in a cardiac muscle tissue can be acquired.

While certain embodiments have been described, these embodiments have been presented by way of example only, and are not intended to limit the scope of the invention as defined by the claims. Indeed, the novel embodiments described herein may be embodied in a variety of other forms; furthermore, various omissions, substitutions and changes in the form of the embodiments described herein may be made without departing from the scope of the invention as defined by the appended claims.

## Claims

1. A magnetic resonance imaging apparatus (100) comprising:
an MRI sequence controller (30) configured to acquire an ECG signal from a subject; a first imaging execution unit (22) configured to perform an initial, segmented time-SLIP sequence comprising a plurality of data acquisition cycles, wherein each data acquisition cycle comprises applying a labeling radio frequency (RF) pulse to blood flowing into a myocardium of the subject, the labelling RF pulse being applied when an initial trigger delay D1 has expired after a detected R-wave of the ECG signal, each data acquisition cycle further comprising acquiring multiple non-contrast magnetic resonance (MR) data sets for which the time intervals between labeling and acquiring data are different by performing a segmented cine sub-sequence of an imaging area including the myocardium, the segmented cine sub-sequence being started after an initial black-blood time to inversion (BBTI) delay interval D4 from the initial trigger delay D1 and having a duration D2, the first imaging execution unit (22) being configured to start a subsequent data acquisition cycle when a next R-wave is detected after a recovery time delay D3 has expired after the end of said duration D2 and not to start the subsequent data acquisition cycle when the next R-wave is detected during the recovery time delay D3;
an identifying unit (22) configured to identify, on the basis of the acquired multiple MR data sets, a time interval taken by the labeled blood to reach a given position in the imaging area; and
a second imaging execution unit (22) configured to perform a further time-SLIP sequence, wherein the identified time interval is used as a BBTI delay interval of said further time-SLIP sequence, said further time-SLIP sequence comprising applying a labeling RF pulse to the blood flowing into the myocardium of the subject, and acquiring non-contrast MR data by imaging the imaging area including the myocardium.

2. The magnetic resonance imaging apparatus according to claim 1, further comprising
a receiver (22) configured to receive a setting of a parameter regarding a given time interval of the segmented cine sub-sequence, wherein
the first imaging execution unit (22) is configured to position the given time interval on a time axis of said initial, segmented time-SLIP sequence in accordance with the received setting of the parameter,
wherein the receiver (22) is configured to receive, as the parameter regarding the given time interval, a setting of the recovery time delay D3.

3. The magnetic resonance imaging apparatus (100) according to claim 1, wherein the first imaging execution unit (22) is configured to perform the segmented cine sub-sequence for a duration D2 including diastole.

4. The magnetic resonance imaging apparatus (100) according to claim 1, wherein the first imaging execution unit (22) is configured to acquire two-dimensional MR data, and the second imaging execution unit (22) is configured to acquire three-dimensional MR data.

5. The magnetic resonance imaging apparatus (100) according to claim 1, wherein the identifying unit (22) is configured to output multiple magnetic resonance angiography (MRA) images generated from the acquired multiple MR data sets to a display and, by receiving an input specifying a given MRA image from an operator, to identify a time interval between labeling and acquiring data corresponding to the specified MRA image as the time interval taken by the labeled blood to reach the given position in the imaging area.

6. The magnetic resonance imaging apparatus (100) according to claim 5, wherein the identifying unit (22) is configured to perform an alignment between the multiple MRA images generated from the acquired multiple MR data sets.

7. The magnetic resonance imaging apparatus (100) according to claim 1, wherein the identifying unit (22) is configured to identify the time interval taken by the labeled blood to reach the given position in the imaging area on the basis of an analysis result of signal values analyzed from the acquired multiple MR data sets.

8. The magnetic resonance imaging apparatus (100) according to claim 1, wherein the identifying unit (22) is configured to output an analysis result of signal values analyzed from the acquired multiple MR data sets and, by receiving an input with respect to the analysis result from an operator, to identify the time interval taken by the labeled blood to reach the given position in the imaging area.

9. A magnetic resonance imaging method performed by a magnetic resonance imaging apparatus, the method comprising:
a step of acquiring an ECG signal from a subject a first imaging execution step of performing an initial, segmented time-SLIP sequence comprising a plurality of data acquisition cycles, wherein each data acquisition cycle comprises
applying a labeling RF pulse to blood flowing into a myocardium of the subject, the labelling RF pulse being applied when an initial trigger delay D1 has expired after a detected R-wave of the ECG signal, each data acquisition cycle further comprising acquiring multiple non-contrast MR data sets for which the time intervals between labeling and acquiring data are different by performing a segmented cine sub-sequence of an imaging area including the myocardium, the segmented cine sub-sequence being started after an initial black-blood time to inversion (BBTI) delay interval D4 from the initial trigger delay D1 and having a duration D2, wherein the first imaging execution step involves to start a subsequent data acquisition cycle when a next R-wave is detected after a recovery time delay D3 has expired after the end of said duration D2 and not to start the subsequent data acquisition cycle when the next R-wave is detected during the recovery time delay D3;
an identifying step of identifying, on the basis of the acquired multiple MR data sets, a time interval taken by the labeled blood to reach a given position in the imaging area; and
a second imaging execution step of performing a further time-SLIP sequence, wherein the identified time interval is used as a BBTI delay interval of said further time-SLIP sequence, said further time-SLIP sequence comprising applying a labeling RF pulse to the blood flowing into the myocardium of the subject, and acquiring non-contrast MR data by imaging the imaging area including the myocardium.

## Patentansprüche

1. Magnetresonanztomographiegerät (100), das Folgendes umfasst:
eine MRI-Sequenzsteuerung (30), konfiguriert zum Erfassen eines EKG-Signals von einem Subjekt;
eine erste Bildgebungsausführungseinheit (22), konfiguriert zum Durchführen einer anfänglichen, segmentierten Zeit-SLIP-Sequenz, die eine Vielzahl von Datenerfassungszyklen umfasst, wobei jeder Datenerfassungszyklus das Anlegen eines Markierungs-Hochfrequenz-(HF)-Impulses an in ein Myokard des Subjekts fließendes Blut umfasst, wobei der Markierungs-HF-Impuls angelegt wird, wenn eine anfängliche Triggerverzögerung D1 nach einer erkannten R-Welle des EKG-Signals abgelaufen ist, wobei jeder Datenerfassungszyklus ferner das Erfassen mehrerer kontrastfreier Magnetresonanz-(MR)-Datensätze, für die die Zeitintervalle zwischen der Markierung und der Erfassung von Daten unterschiedlich sind, durch Durchführen einer segmentierten Cine-Subsequenz eines das Myokard einschließenden Bildgebungsbereichs umfasst, wobei die segmentierte Cine-Subsequenz nach einem anfänglichen BBTI-(Black-Blood Time to Inversion)-Verzögerungsintervall D4 ab der anfänglichen Triggerverzögerung D1 gestartet wird und eine Dauer D2 hat,
wobei die erste Bildgebungsausführungseinheit (22) zum Starten eines nachfolgenden Datenerfassungszyklus, wenn eine nächste R-Welle nach Ablauf einer Erholungszeitverzögerung D3 nach dem Ende der Dauer D2 erkannt wird, und zum Nichtstarten des nachfolgenden Datenerfassungszyklus konfiguriert ist, wenn die nächste R-Welle während der Erholungszeitverzögerung D3 erkannt wird;
eine Identifizierungseinheit (22), konfiguriert zum Identifizieren, auf der Basis der erfassten mehreren MR-Datensätze, eines Zeitintervalls, das das markierte Blut benötigt, um eine gegebene Position in dem Bildgebungsbereich zu erreichen; und
eine zweite Bildgebungsausführungseinheit (22), konfiguriert zum Durchführen einer weiteren Zeit-SLIP-Sequenz, wobei das identifizierte Zeitintervall als BBTI-Verzögerungsintervall der weiteren Zeit-SLIP-Sequenz verwendet wird, wobei die weitere Zeit-SLIP-Sequenz das Anlegen eines Markierungs-HF-Impulses an das in das Myokard des Subjekts fließende Blut und das Erfassen von kontrastfreien MR-Daten durch Abbilden des Bildgebungsbereichs einschließlich des Myokards umfasst.

2. Magnetresonanztomographiegerät nach Anspruch 1, das ferner Folgendes umfasst:
einen Empfänger (22), konfiguriert zum Empfangen einer Einstellung eines Parameters bezüglich eines gegebenen Zeitintervalls der segmentierten Cine-Subsequenz, wobei
die erste Bildgebungsausführungseinheit (22) zum Positionieren des gegebenen Zeitintervalls auf einer Zeitachse der anfänglichen, segmentierten Zeit-SLIP-Sequenz gemäß der empfangenen Einstellung des Parameters konfiguriert ist,
wobei der Empfänger (22) zum Empfangen einer Einstellung der Erholungszeitverzögerung D3 als Parameter bezüglich des gegebenen Zeitintervalls konfiguriert ist.

3. Magnetresonanztomographiegerät (100) nach Anspruch 1, wobei die erste Bildgebungsausführungseinheit (22) zum Ausführen der segmentierten Cine-Subsequenz für eine Dauer D2 einschließlich Diastole konfiguriert ist.

4. Magnetresonanztomographiegerät (100) nach Anspruch 1, wobei
die erste Bildgebungsausführungseinheit (22) zum Erfassen zweidimensionaler MR-Daten konfiguriert ist, und
die zweite Bildgebungsausführungseinheit (22) zum Erfassen dreidimensionaler MR-Daten konfiguriert ist.

5. Magnetresonanztomographiegerät (100) nach Anspruch 1, wobei die Identifizierungseinheit (22) zum Ausgeben mehrerer aus den erfassten mehreren MR-Datensätzen erzeugter MRA-(Magnet Resonance Angiography)-Bilder an ein Display und zum Identifizieren, durch Empfangen einer ein gegebenes MRA-Bild von einem Bediener spezifizierenden Eingabe, eines Zeitintervalls zwischen der Markierung und der Erfassung von Daten entsprechend dem spezifizierten MRA-Bild als das Zeitintervall konfiguriert ist, das das markierte Blut benötigt, um die gegebene Position in dem Bildgebungsbereich zu erreichen.

6. Magnetresonanztomographiegerät (100) nach Anspruch 5, wobei die Identifizierungseinheit (22) zum Durchführen einer Ausrichtung zwischen den mehreren aus den erfassten mehreren MR-Datensätzen erzeugten MRA-Bildem konfiguriert ist.

7. Magnetresonanztomographiegerät (100) nach Anspruch 1, wobei die Identifizierungseinheit (22) zum Identifizieren des Zeitintervalls, das das markierte Blut benötigt, um die gegebene Position im Bildgebungsbereich zu erreichen, auf der Basis eines Analyseergebnisses von aus den erfassten mehreren MR-Datensätzen analysierten Signalwerten konfiguriert ist.

8. Magnetresonanztomographiegerät (100) nach Anspruch 1, wobei die Identifizierungseinheit (22) zum Ausgeben eines Analyseergebnisses von aus den erfassten mehreren MR-Datensätzen analysierten Signalwerten und zum Identifizieren, durch Empfangen einer Eingabe bezüglich des Analyseergebnisses von einem Bediener, des Zeitintervalls konfiguriert ist, das das markierte Blut benötigt, um die gegebene Position in dem Bildgebungsbereich zu erreichen.

9. Magnetresonanzbildgebungsverfahren, das von einem Magnetresonanztomographiegerät durchgeführt wird, wobei das Verfahren Folgendes umfasst:
einen Schritt des Erfassens eines EKG-Signals von einem Subjekt,
einen ersten Bildgebungsausführungsschritt des Durchführens einer anfänglichen, segmentierten Zeit-SLIP-Sequenz, die eine Vielzahl von Datenerfassungszyklen umfasst, wobei jeder Datenerfassungszyklus das Anlegen eines Markierungs-HF-Impulses an in ein Myokard des Subjekts fließendes Blut umfasst, wobei der Markierungs-HF-Impuls angelegt wird, wenn eine anfängliche Triggerverzögerung D1 nach einer detektierten R-Welle des EKG-Signals abgelaufen ist, wobei jeder Datenerfassungszyklus ferner das Erfassen mehrerer kontrastfreier MR-Datensätze, für die die Zeitintervalle zwischen der Markierung und der Erfassung von Daten unterschiedlich sind, durch Durchführen einer segmentierten Cine-Subsequenz eines das Myokard einschließenden Bildgebungsbereichs umfasst, wobei die segmentierte Cine-Subsequenz nach einem anfänglichen BBTI-(Black-Blood Time to Inversion)-Verzögerungsintervall D4 ab der anfänglichen Triggerverzögerung D1 gestartet wird und eine Dauer D2 hat, wobei der erste Bildgebungsausführungsschritt das Starten eines nachfolgenden Datenerfassungszyklus, wenn eine nächste R-Welle nach Ablauf einer Erholungszeitverzögerung D3 nach dem Ende der Dauer D2 ist erkannt wird, und das Nichtstarten des nachfolgenden Datenerfassungszyklus beinhaltet, wenn die nächste R-Welle während der Erholungszeitverzögerung D3 erkannt wird;
einen Identifizierungsschritt des Identifizierens eines Zeitintervalls, das das markierte Blut benötigt, um eine gegebene Position in dem Bildgebungsbereich zu erreichen, auf der Basis der erfassten mehreren MR-Datensätze; und
einen zweiten Bildgebungsausführungsschritt des Durchführens einer weiteren Zeit-SLIP-Sequenz, wobei das identifizierte Zeitintervall als ein BBTI-Verzögerungsintervall der weiteren Zeit-SLIP-Sequenz verwendet wird, wobei die weitere Zeit-SLIP-Sequenz das Anlegen eines Markierungs-HF-Impulses an das in das Myokard des Subjekts fließende Blut und das Erfassen von kontrastfreien MR-Daten durch Bildgebung des Bildgebungsbereichs einschließlich des Myokards umfasst.

## Revendications

1. Appareil d'imagerie par résonance magnétique (100) comprenant :
un contrôleur de séquences d'IRM (30) configuré pour acquérir un signal d'ECG à partir d'un sujet ;
une première unité d'exécution d'imagerie (22) configurée pour réaliser une séquence temps-SLIP segmentée initiale comprenant une pluralité de cycles d'acquisition de données, dans lequel chaque cycle d'acquisition de données comprend l'application d'une impulsion radiofréquence (RF) d'étiquetage sur du sang qui circule à l'intérieur d'un myocarde du sujet, l'impulsion RF d'étiquetage étant appliquée lorsqu'un retard de déclenchement initial D1 a expiré après une onde R détectée du signal d'ECG, chaque cycle d'acquisition de données comprenant en outre l'acquisition de plusieurs ensembles de données de résonance magnétique (RM) sans contraste pour lesquels les intervalles temporels entre l'étiquetage et l'acquisition de données sont différents en réalisant une sous-séquence ciné segmentée d'une zone d'imagerie incluant le myocarde, la sous-séquence ciné segmentée étant démarrée après un intervalle de retard de temps de sang noir jusqu'à inversion (BBTI) initial D4 décompté à partir du retard de déclenchement initial D1 et présentant une durée D2,
la première unité d'exécution d'imagerie (22) étant configurée pour démarrer un cycle d'acquisition de données subséquent lorsqu'une onde R suivante est détectée après qu'un retard de temps de restauration D3 a expiré après la fin de ladite durée D2 et pour ne pas démarrer le cycle d'acquisition de données subséquent lorsque l'onde R suivante est détectée pendant le retard de temps de restauration D3 ;
une unité d'identification (22) configurée pour identifier, sur la base des plusieurs ensembles de données de RM acquis, un intervalle temporel nécessaire pour que le sang étiqueté atteigne une position donnée dans la zone d'imagerie ; et
une deuxième unité d'exécution d'imagerie (22) configurée pour réaliser une autre séquence temps-SLIP, dans lequel l'intervalle temporel identifié est utilisé en tant qu'intervalle de retard de BBTI de ladite autre séquence temps-SLIP, ladite autre séquence temps-SLIP comprenant l'application d'une impulsion RF d'étiquetage sur le sang qui circule à l'intérieur du myocarde du sujet, et l'acquisition de données de RM sans contraste en imageant la zone d'imagerie incluant le myocarde.

2. Appareil d'imagerie par résonance magnétique selon la revendication 1, comprenant en outre :
un récepteur (22) configuré pour recevoir un réglage d'un paramètre concernant un intervalle temporel donné de la sous-séquence ciné segmentée, dans lequel
la première unité d'exécution d'imagerie (22) est configurée pour positionner l'intervalle temporel donné sur un axe des temps de ladite séquence temps-SLIP segmentée initiale en fonction du réglage reçu du paramètre,
dans lequel le récepteur (22) est configuré pour recevoir, en tant que paramètre concernant l'intervalle temporel donné, un réglage du retard de temps de restauration D3.

3. Appareil d'imagerie par résonance magnétique (100) selon la revendication 1, dans lequel la première unité d'exécution d'imagerie (22) est configurée pour réaliser la sous-séquence ciné segmentée pendant une durée D2 incluant une diastole.

4. Appareil d'imagerie par résonance magnétique (100) selon la revendication 1, dans lequel
la première unité d'exécution d'imagerie (22) est configurée pour acquérir des données de RM bidimensionnelles, et
la deuxième unité d'exécution d'imagerie (22) est configurée pour acquérir des données de RM tridimensionnelles.

5. Appareil d'imagerie par résonance magnétique (100) selon la revendication 1, dans lequel l'unité d'identification (22) est configurée pour émettre en sortie plusieurs images d'angiographie par résonance magnétique (ARM) générées à partir des plusieurs ensembles de données de RM acquis sur un affichage et pour identifier, par la réception d'une entrée spécifiant une image d'ARM donnée en provenance d'un opérateur, un intervalle temporel entre l'étiquetage et l'acquisition de données correspondant à l'image d'ARM spécifiée en tant qu'intervalle temporel nécessaire pour que le sang étiqueté atteigne la position donnée dans la zone d'imagerie.

6. Appareil d'imagerie par résonance magnétique (100) selon la revendication 5, dans lequel l'unité d'identification (22) est configurée pour réaliser un alignement entre les plusieurs images d'ARM générées à partir des plusieurs ensembles de données de RM acquis.

7. Appareil d'imagerie par résonance magnétique (100) selon la revendication 1, dans lequel l'unité d'identification (22) est configurée pour identifier l'intervalle temporel nécessaire pour que le sang étiqueté atteigne la position donnée dans la zone d'imagerie sur la base d'un résultat d'analyse de valeurs de signal analysées à partir des plusieurs ensembles de données de RM acquis.

8. Appareil d'imagerie par résonance magnétique (100) selon la revendication 1, dans lequel l'unité d'identification (22) est configurée pour émettre en sortie un résultat d'analyse de valeurs de signal analysées à partir des plusieurs ensembles de données de RM acquis et pour identifier, par la réception d'une entrée en relation avec le résultat d'analyse en provenance d'un opérateur, l'intervalle temporel nécessaire pour que le sang étiqueté atteigne la position donnée dans la zone d'imagerie.

9. Procédé d'imagerie par résonance magnétique réalisé par un appareil d'imagerie par résonance magnétique, le procédé comprenant :
une étape d'acquisition d'un signal d'ECG à partir d'un sujet ;
une première étape d'exécution d'imagerie consistant à réaliser une séquence temps-SLIP segmentée initiale comprenant une pluralité de cycles d'acquisition de données, dans lequel chaque cycle d'acquisition de données comprend l'application d'une impulsion RF d'étiquetage sur du sang qui circule à l'intérieur d'un myocarde du sujet, l'impulsion RF d'étiquetage étant appliquée lorsqu'un retard de déclenchement initial D1 a expiré après une onde R détectée du signal d'ECG, chaque cycle d'acquisition de données comprenant en outre l'acquisition de plusieurs ensembles de données de RM sans contraste pour lesquels les intervalles temporels entre l'étiquetage et l'acquisition de données sont différents en réalisant une sous-séquence ciné segmentée d'une zone d'imagerie incluant le myocarde, la sous-séquence ciné segmentée étant démarrée après un intervalle de retard de temps de sang noir jusqu'à inversion (BBTI) initial D4 décompté à partir du retard de déclenchement initial D1 et présentant une durée D2, dans lequel la première étape d'exécution d'imagerie implique le démarrage d'un cycle d'acquisition de données subséquent lorsqu'une onde R suivante est détectée après qu'un retard de temps de restauration D3 a expiré après la fin de ladite durée D2 et le non démarrage du cycle d'acquisition de données subséquent lorsque l'onde R suivante est détectée pendant le retard de temps de restauration D3 ;
une étape d'identification consistant à identifier, sur la base des plusieurs ensembles de données de RM acquis, un intervalle temporel nécessaire pour que le sang étiqueté atteigne une position donnée dans la zone d'imagerie ; et
une deuxième étape d'exécution d'imagerie consistant à réaliser une autre séquence temps-SLIP, dans lequel l'intervalle temporel identifié est utilisé en tant qu'intervalle de retard de BBTI de ladite autre séquence temps-SLIP, ladite autre séquence temps-SLIP comprenant l'application d'une impulsion RF d'étiquetage sur le sang qui circule à l'intérieur du myocarde du sujet, et l'acquisition de données de RM sans contraste en imageant la zone d'imagerie incluant le myocarde.
